# EUROPÄISCHE PATENTSCHRIFT

(11) **EP 0 434 618 B1**
(45) Veröffentlichungstag und Bekanntmachung des Hinweises auf die Patenterteilung: **05.04.1995**
(21) Anmeldenummer: 90810977.0
(22) Anmeldetag: 12.12.1990
(51) Int. Cl.: C07D 311/86, C07D 219/06, C08K 5/25, C08K 5/15

(54) **Uv-Absorber und diese enthaltendes lichtempfindliches organisches Material**
UV-absorber and light sensible organic material containing it
Absorbeur d'UV et matériel organique sensible à la lumière le contenant

(30) Priorität: 21.12.1989 CH 4579/89
(43) Veröffentlichungstag der Anmeldung: 26.06.1991
(73) Patentinhaber: CIBA-GEIGY AG, 4002 Basel (CH)
(72) Erfinder: Berner, Godwin, Dr., CH-4102 Binningen (CH); Valet, Andreas, Dr., W-7859 Eimeldingen (DE)

(56) Entgegenhaltungen:
- CH-A- 379 760
- CH-A- 510 046
- MONATSHEFTE FÜR CHEMIE, Band 94, Nr. 2, 1963, Seiten 410-413, Wien, AT; G.S.PURANIK et al.: "Selektive Alkylierung chelatisierter Hydroxygruppen in Kondensierten Benzpyronen, 1. Mitt."
- CHEMICAL ABSTRACTS, Band 110, Nr. 4, 23. Januar 1989, Seite 503, Zusammenfassung Nr. 31366t, Columbus, Ohio, US; & JP-A-63 155 048 (KONICA) 28-06-1988
- INDIAN JOURNAL OF CHEMISTRY, Band 10, Nr. 7, Juli 1972, Seiten 1157-1158, NewDelhi, IN; G.S. PURANIK et al.: "Formylation of hydroxyxanthones"
- HELVETICA CHIMICA ACTA, Band 62, Nr. 3, 20. April 1979, Seiten 678-682, Basel,CH; V.M. CHARI et al.: "Synthesis of xanthone-O-glycosides. II. Synthesis of 1-O-beta-glycosides"
- BULLETIN DES SOCIETES CHIMIQUES BELGES, Band 93, Nr. 3, 1984, Seiten 239-240,Oxford, GB; R.R. SMOLDERS et al.: "Synthesis of some hydroxylated 9-acridanones"
- AUSTRALIAN JOURNAL OF CHEMISTRY, Band 23, Nr. 9, September 1970, Seiten 1881-1889, Melbourne, AU; J. HLUBUCEK et al.: "A synthesis of acronycine"
- TETRAHEDRON, Band 25, Nr. 2, 1969, Seiten 275-282, Oxford, GB; A.C. JAIN et al.: "Synthesis and study of 2-C- and 4-C-methylxanthones"

## Beschreibung

Die vorliegende Erfindung betrifft neue UV-Absorber vom Xanthontyp und diese enthaltendes lichtempfindliches organisches Material.

In CH-A-379,760 wird ein Verfahren zum Schützen lichtempfindlicher Materialien vor der schädlichen Einwirkung von Licht, insbesondere von UV-Strahlen, beschrieben, worin 1-Hydroxyxanthonverbindungen Anwendung finden.

Xanthonverbindungen, welche einen unsubstituierten Alkoxyrest aufweisen, sind beispielsweise aus Monatshefte für Chemie 94, 410-413 (1963), Chemical Abstracts, 110, 31366t (1989), Helvetica Chimica Acta, 62, 678-682 (1979), Bulletin des Sociétés Belges, 93, 239-240 (1984), Australien Journal of Chemistry, 23, 1881-1889 (1970) und Tetrahedron, 25, 275-282 (1970) bekannt. Aus dem Indian Journal of Chemistry ist die Verbindung 1-Hydroxy-3-O-carboethoxymethylxanthon bekannt.

Es wurde nun gefunden, dass eine weitere Klasse von Xanthonverbindungen mit gutem Erfolg für diesen Zweck eingesetzt werden können.

Gegenstand der vorliegenden Erfindung sind Verbindungen der Formel 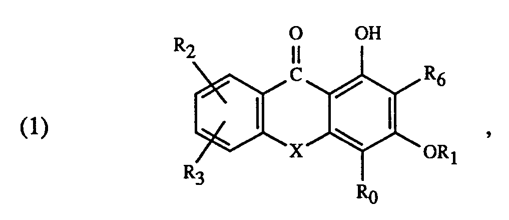
worin X O und R₀ Wasserstoff oder ein Rest der Formel -(CH₂)ₙCO₂R ist, worin n 1 oder 2 und R Alkyl mit 1 bis 18 Kohlenstoffatomen oder 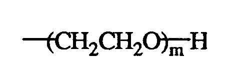
ist, worin m 1 bis 12 ist, R₁ mit Hydroxyl substituiertes und mit Sauerstoff unterbrochenes Alkyl mit 4 bis 18 Kohlenstoffatomen, -COR₄, worin R₄ Alkyl oder Alkenyl mit je 2 bis 12 Kohlenstoffatomen, 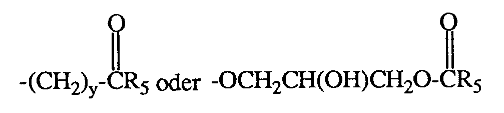
ist, oder R₁ 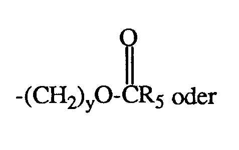 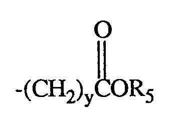
bedeutet, wobei R₅ Alkyl mit 1 bis 12 Kohlenstoffatomen oder Alkenyl mit 2 bis 12 Kohlenstoffatomen und y 1 bis 12 ist, und
R₂ und R₃ unabhängig voneinander Wasserstoff, Alkyl mit 1 bis 12 Kohlenstoffatomen, Alkenyl mit 2 bis 12 Kohlenstoffatomen, -OR₁, worin R₁ die angegebene Bedeutung hat, oder Chlor sind, und R₆ Wasserstoff oder Alkyl mit 1 bis 4 Kohlenstoffatomen ist, ausgenommen die Verbindung, worin X O, R₀, R₂, R₃ und R₆ Wasserstoff und R₁ -CH₂-C(O)OC₂H₅ bedeutet.

Gegenstand der vorliegenden Erfindung sind ferner ein Verfahren zur Herstellung dieser Verbindungen, lichtempfindliches organisches Material, das mindestens eine dieser Verbindungen enthält, sowie ein Verfahren zum Schützen lichtempfindlicher organischer Materialien durch Verwendung der Verbindungen der Formel (1).

In den Verbindungen der Formel (1) bedeutet der Substituent R₀ neben Wasserstoff einen Rest der Formel -(CH₂)ₙ-CO₂R. Darin ist R ein Alkylrest mit 1 bis 18 Kohlenstoffatomen, wie z.B. Methyl, Äthyl, Propyl, Butyl, Hexyl, Octyl, Decyl, Undecyl, Dodecyl, Hexadecyl und Octadecyl sowie entsprechende verzweigte Isomere, oder ein Rest der Formel 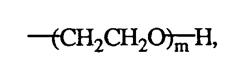
worin m eine ganze Zahl von 1 bis 12 ist. Index n ist 1 oder 2. Die Alkylreste R₁ enthalten 4 bis 18 Kohlenstoffatome und sind mit einer oder mehreren Hydroxylgruppen substituiert und auch durch ein oder mehrere Sauerstoffatome unterbrochen. Als Beispiele solcher Alkylreste wären Gruppierungen wie 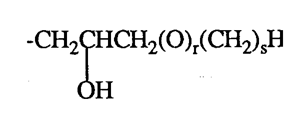
zu nennen, worin r 1 und s 1 bis 15 ist.

R₁ bedeutet ferner einen Rest der Formel -COR₄, worin R₄ Alkyl oder Alkenyl mit je 2 bis 12 Kohlenstoffatomen ist Beispiele für solche Alkylreste sind oben genannt. Beispiele für die Alkenylreste sind Aethenyl, Propenyl, Butenyl, Hexenyl, Octenyl, Nonenyl und Dodecenyl, wobei die Alkenylreste R₁ auch mehrfach ungesättigt sein können. Auch kann R₁ für die entsprechenden verzweigten Isomeren stehen. R₄ bedeutet auch Reste der Formeln 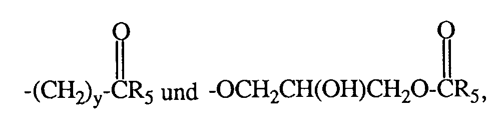
worin R₅ Alkyl mit 1 bis 12 Kohlenstoffatomen oder Alkenyl mit 2 bis 12 Kohlenstoffatomen (Beispiele siehe oben) und y 1 bis 12 ist.

Des weiteren bedeutet R₁ einen Rest der Formel 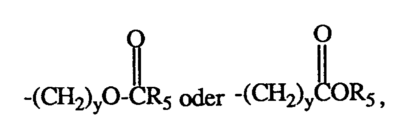
worin R₅ und y die angegebene Bedeutung haben.

Die Substituenten R₂ und R₃ bedeuten unabhängig voneinander Wasserstoff, Alkyl mit 1 bis 12 Kohlenstoffatomen oder Alkenyl mit 2 bis 12 Kohlenstoffatomen (Beispiele siehe oben), OR₁, worin R₁ die angegebene Bedeutung hat, oder Chlor.

X ist ein zweiwertiger Rest und bedeutet O.

Vorzugsweise sind in den Verbindungen der Formel (1) die Substituenten R₂ und R₃ Wasserstoff oder Methyl.

Eine weitere Gruppe bevorzugten Verbindungen der Formel (1) ist dadurch gekennzeichnet, dass R₁ mit Hydroxyl substituiertes und durch Sauerstoff unterbrochenes Alkyl mit 4 bis 12 Kohlenstoffatomen -COR₄ oder 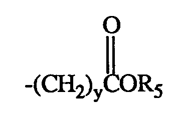
ist, worin R₄ Alkyl mit 4 bis 8 Kohlenstoffatomen, y 1 bis 4 und R₅ Alkyl mit 1 bis 4 Kohlenstoffatomen ist.

Des weiteren sind solche Verbindungen der Formel (1) bevorzugt, worin R₀ ein Rest der Formel -CH₂CH₂CO₂R ist, worin R Alkyl mit 1 bis 8 Kohlenstoffatomen oder 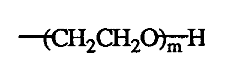
ist und m 6 bis 8 ist. Lichtempfindliche organische Materialien können erfindungsgemäss vor dem schädigenden Einfluss von UV-Strahlung geschützt werden, indem man diese Materialien mit einer Schutzschicht, z.B. einem Lack, versieht, die mindestens eine Verbindung der Formel (1) enthält, oder eine solche Verbindung auf an sich übliche Weise in das organische Material einarbeitet.

Lichtempfindliche organische Materialien sind erfindungsgemäss frei von sterisch gehinderten Aminen oder Hydroxyphenylbenztriazolderivaten.

Beispiele für lichtempfindliche organische Materialien, die erfindungsgemäss vor schädigender Lichteinwirkung geschützt werden können, sind:
1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Kohlenwasserstoffharze (z.B. C₅-C₉) inklusive hydrierte Modifikationen davon (z.B. Klebrigmacherharze).
4. Polystyrol, Poly-(p-methylstyrol), Poly-(α-methylstyrol).
5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von α,β-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherketone.
20. Vernetzte Polymere, die sich von Aldehyden einerseits und Phenolen, Harnstoff oder Melamin andererseits ableiten, wie Phenol-Formaldehyd, Harnstoff-Formaldehyd- und Melamin-Formaldehydharze.
21. Trocknende und nicht-trocknende Alkydharze.
22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten, wie z.B. von Epoxyacrylaten, Urethan-acrylaten oder Polyester-acrylaten.
24. Alkydharze, Polyesterharze und Acrylatharze, die mit Melaminharzen, Harnstoffharzen, Polyisocyanaten oder Epoxidharzen vernetzt sind.
25. Vernetzte Epoxidharze, die sich von Polyepoxiden ableiten, z.B. von Bis-glycidylethern oder von cycloaliphatischen Diepoxiden.
26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Die erfindungsgemässen lichtempfindlichen organischen Materialien können neben den Verbindungen der Formel (1) auch weitere übliche Stabilisatoren und Zusätze enthalten, wie z.B.:
1. Antioxidantien
   1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.
   1.2. Alkylierte Hydrochinone, z.B. 2,6-Di-tert.butyl-4-methoxyphenol, 2,5-Di-tert.butylhydrochinon, 2,5-Di-tert.amyl-hydrochinon, 2,6-Diphenyl-4-octadecyloxyphenol.
   1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).
   1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol-bis-[3,3-bis-(3'-tert.-butyl-4'-hydroxyphenyl)-butyrat], Bis-(3-tert.butyl-4-hydroxy-5-methyl-phenyl)-dicyclopentadien, Bis-[2-(3'-tert.butyl-2'-hydroxy-5'-methyl-benzyl)-6-tert.butyl-4-methyl-phenyl]-terephthalat.
   1.5 Benzylverbindungen, z.B. 1,3,5-Tris-(3,5-di-tert.butyl-4-hydroxy-benzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.
   1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino )-s-triazin, N-(3,5-di-tert.butyl-4-hydroxyphenyl)-carbaminsäureoctylester.
   1.7. Ester der β-(3,5-Di-tert.butyl-4-hydroxyphenyl)-propionsäure mit ein- oder mehrwertigen Alkoholen, wie z.B. mit Methanol, Octa- decanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.
   1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.
   1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.
   1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.
2. UV-Absorber und Lichtschutzmittel
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.
   2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.
   2.7. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)- 1,3,5-triazin, 2-(2-Hydroxy-4-dodecyloxyphenyl)-4,6-bis(2,4-dimethylphenyl)-1,3,5-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazi n, 3-Salicyloylamino-1,2,4-triazol, Bis-(benzyliden)-oxalsäuredihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.
8. Nukleierungsmittel, wie z.B. 4-tert.Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.
10. Sonstige Zusätze, wie z.B. Weichmacher, Gleitmittel, Emulgatoren, Pigmente, Optische Aufheller, Flammschutzmittel, Antistatika, Treibmittel.

Von den organischen Materialien, die erfindungsgemäss gegen Lichtschädigung geschützt werden können, sind organische Polymere hervorzuheben, insbesondere synthetische Polymere. Bevorzugt werden daher thermoplastische Polymere und insbesondere Lackzusammensetzungen geschützt, wobei bei letzteren insbesondere der polymere Binder gegen Lichteinfluss stabilisiert wird.

Lackzusammensetzungen bzw. Lacke, welche die Verbindungen der Formel (1) enthalten, können z.B. pigmentierte oder unpigmentierte Lacke oder Metalleffektlacke sein. Sie können ein organisches Lösungsmittel enthalten oder lösungsmittelfrei sein oder wässrige Lacke sein.

Die Lacke können als Bindemittel mindestens eines der vorhin aufgeführten Polymeren enthalten. Beispiele für Lacke mit speziellen Bindemitteln sind die folgenden:
1. Lacke auf Basis von kalt- oder heiss-vernetzbaren Alkyd-, Acrylat-, Polyester-, Epoxid- oder Melaminharzen oder Mischungen solcher Harze, gegebenenfalls mit Zusatz eines sauren Härtungskatalysators;
2. Zweikomponenten-Polyurethanlacke auf Basis von hydroxylgruppenhaltigen Acrylat-, Polyester- oder Polyetherharzen und aliphatischen oder aromatischen Polyisocyanaten;
3. Einkomponenten-Polyurethanlacke auf Basis von blockierten Polyisocyanaten, die während des Einbrennens deblockiert werden;
4. Zweikomponentenlacke auf Basis von (Poly)ketiminen und aliphatischen oder aromatischen Polyisocyanaten;
5. Zweikomponentenlacke auf Basis von (Poly)ketiminen und einem ungesättigten Acrylatharz oder einem Polyacetoacetatharz oder einem Methylacrylamidoglykolatmethylester;
6. Zweikomponentenlacke auf Basis von carboxyl- oder aminogruppenhaltigen Polyacrylaten und Polyepoxiden;
7. Zweikomponentenlacke auf Basis von anhydridgruppenhaltigen Acrylatharzen und einer Polyhydroxy- oder Polyaminokomponente;
8. Zweikomponentenlacke auf Basis von (Poly)oxazolidinen und anhydridgruppenhaltign Acrylatharzen oder ungesättigten Acrylatharzen oder aliphatischen oder aromatischen Polyisocyanaten.
9. Zweikomponentenlacke auf Basis von ungesättigten Polyacrylaten und Polymalonaten;
10. thermoplastische Polyacrylatlacke auf Basis von thermoplastischen Acrylatharzen oder fremdvernetzenden Acrylatharzen in Kombination mit veretherten Melaminharzen;
11. Lacksysteme auf Basis von siloxanmodifizierten Acrylatharzen; und
12. Lacksysteme auf Basis von fluormodifizierten Acrylatharzen.

Die Lacke können auch strahlenhärtbare Lacke sein. In diesem Fall besteht das Bindungsmittel aus monomeren oder oligomeren Verbindungen, die ethylenische Doppelbindungen enthalten und durch Bestrahlung mit aktinischem Licht oder mit Elektronenstrahlen in eine vernetzte hochmolekulare Form übergehen. Meist handelt es sich hierbei um ein Gemisch solcher Verbindungen.

Die Lacke können als Einschicht- oder Zweischichtlacke appliziert werden, wobei die erfindungsgemässen Stabilisatoren vorzugsweise der obersten Schicht zugesetzt werden.

Die Lacke können auf die Substrate (Metall, Plastik, Holz etc.) nach den üblichen Verfahren aufgebracht werden, beispielsweise durch Streichen, Besprühen, Giessen, Tauchen oder Elektrophorese.

Besonders bevorzugt sind Lacke, die auf Automobile appliziert werden (Autolacke). Sofern es sich bei den erfindungsgemässen lichtempfindlichen organischen Materialien um Lackzusammensetzungen handelt, enthalten letztere bevorzugt keine Alkydharze als Binder.

Die Verbindungen der Formel (1) können nach üblichen, bekannten Methoden in die Lacke bzw. Lackzusammensetzungen sowie andere lichtempfindliche organische Materialien eingearbeitet werden. In der Regel betragen die Mengenverhältnisse 0,01 bis 5,0, insbesondere 0,02 bis 3,0 Gew.-% UV-Absorber, bezogen auf das organische Material, wobei die zu wählenden Mengen an UV-Absorber von der Natur des Materials und den Anforderungen an dessen Stabilität abhängig sind.

Die Komponenten von Stabilisatorkombinationen können einzeln oder als Gemisch dem organischen Material zugesetzt werden. Der Zusatz der Verbindungen der Formel (1) und gegebenenfalls weiterer Stabilisatoren erfolgt vorzugsweise vor oder während der Formgebung des Materials, sofern es sich z.B. um thermoplastische Kunststoffe handelt. Die Zugabe kann jedoch auch bereits während der Polymerisation oder vor dieser zu den Ausgangsmonomeren erfolgen.

Die Verbindungen der Formel (1) lassen sich nach an sich bekannten Methoden herstellen. Beispielsweise kann durch Erhitzen eines Gemisches aus den Verbindungen der Formeln 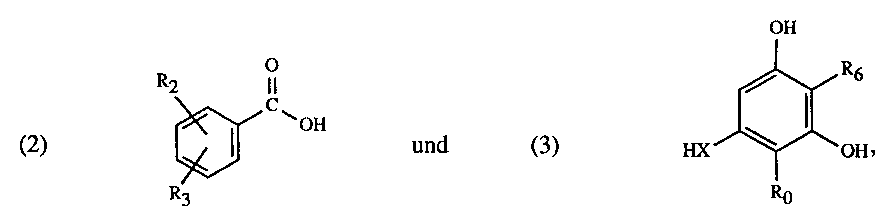
worin R₀, R₂, R₃, R₆ und X die angegebene Bedeutung haben, die Zwischenverbindung der Formel 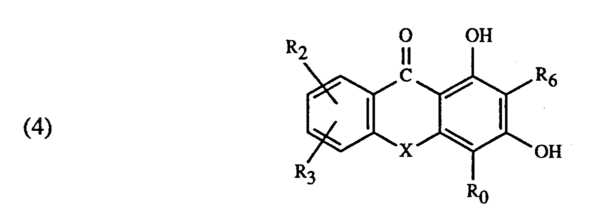
erhalten werden, welche sich durch Umsetzung mit beispielsweise R₁-Cl oder R₁-Br, worin R₁ die angegebene Bedeutung hat, in die Verbindung der Formel (1) überführen lässt.

Die folgenden Beispiele erläutern die Erfindung weiter. Angaben in Teilen oder Prozenten beziehen sich - sofern nicht anders angegeben - auf das Gewicht.

### Herstellungsbeispiele:

Beispiel 1: 22,8 g 1,3-Dihydroxyxanthon (hergestellt nach Grover, J. Chem. Soc. 1955, 3982) werden in 300 ml absolutem Aethanol vorgelegt. Dazu gibt man langsam, in Portionen, 11,2 g Kalium-tert.-butylat und dann 0,5 g Kaliumjodid. Bei Raumtemperatur werden in ca. 15 Minuten 12,3 g Chloressigsäure-äthylester zugetropft und das Reaktionsgemisch anschliessend 6 Stunden am Rückfluss erhitzt. Man giesst in 500 ml Wasser und säuert mit 20 ml Eisessig an. Der entstandene Niederschlag wird abgerutscht, getrocknet. Durch Umkristallisation aus Hexan erhält man die Verbindung der Formel 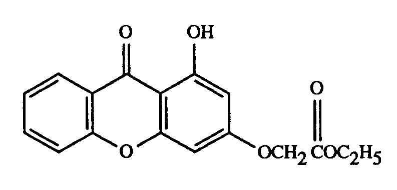
mit einem Schmelzpunkt von 165°C.

Beispiel 2: 22,8 g 1,3-Dihydroxyxanthon (hergestellt nach Grover, J. Chem. Soc. 1955, 3982) werden in 200 ml Xylol auf 120°C erwärmt. Nach der Zugabe von 1,5 g Tetrabutylammoniumbromid werden in ca. 10 Minuten 20,5 g 2-Aethylhexyl-glycidyläther zugetropft und das Reaktionsgemisch dann 24 Stunden bei 120° gerührt. Man gibt dann 5 g Tonsil AC zur Reaktionslösung, rührt 5 Minuten bei 110° und filtriert rein. Das Filtrat wird eingedampft, der Rückstand in 300 ml Hexan aufgenommen und 50 g über Kieselgel filtriert. Nach dem Eindampfen erhält man die Verbindung der Formel 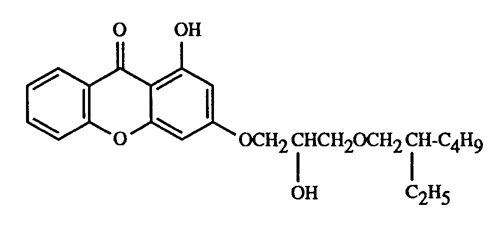
in Form eines gelblichen Harzes, das langsam kristallisiert Der Schmelzpunkt beträgt 56 bis 58°C.

Anwendungsbeispiel 1: Die erfindungsgemässen UV-Absorber werden in einem 2-Schicht Metallic Lack geprüft.

Es wird ein Klarlack der folgenden Zusammensetzung hergestellt:

| | |
|---|---|
| Uracron® 2263 XB (50%) | 59,2 Teile |
| Cymel® 327 (90 %) | 11,6 Teile |
| Baysilon® A (1 % in Xylol) | 1,0 Teile |
| Butylglykolacetat | 5,5 Teile |
| Xylol | 19,4 Teile |
| Butanol | 3,3 Teile |
| | 100,0 Teile |

Dazu wird 1 Teil der Verbindung nach Herstellungsbeispiel 1 bzw. 2, vorgelöst in 10 Teilen Xylol, zugegeben.

Dieser Klarlack wird mit einem Gemisch aus Xylol (13 Teile), Butanol (6 Teile) und Butylglykolacetat (1 Teil) auf Spritzfähigkeit verdünnt und auf ein vorbereitetes Aluminiumblech (coil coat-beschichtet, Automobilfüller, silbermetallic Basislack auf Basis Polyester/Celluloseacetatobutyrat/Melaminharz) gespritzt und bei 130°C 30 Minuten eingebrannt. Es resultiert eine Trockenschichtdicke von 40 bis 50 »m Klarlack. Vergleichsweise wird ein gleichermassen vorbereitetes Aluminiumblech mit dem oben definierten Klarlack, der jedoch frei von UV-Absorbern ist, beschichtet.

Die so erhaltenen Proben werden nach Bewitterung auf Glanzhaltung geprüft. Im Vergleich zur Probe ohne UV-Absorber zeigen die erfindungsgemäss stabilisierten Proben eine deutlich bessere Bewitterungsstabilität. Die Ergebnisse sind in der folgenden Tabelle 1 zusammengestellt.

**Tabelle 1**

| Bewertung der Glanzhaltung nach Bewitterung gemäss DIN 67530 (20°C Glanz) | | | | |
|---|---|---|---|---|
| UV-Absorber | 20° Glanz nach | | | |
| | 0 | 400 | 800 | 1200 Stunden |
| keiner | 86 | 75 | 45 | 16 |
| gemäss Beispiel 1 | 85 | 75 | 68 | 74 |
| gemäss Beispiel 2 | 86 | 80 | 80 | 80 |
| Zyklus: 8 Stunden UV (UVB-313), 70°C; 4 Stunden Kond., 50°C (UVCON) | | | | |

## Patentansprüche

1. Verbindungen der Formel 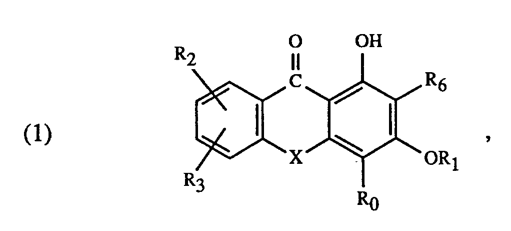 worin X O ist und R₀ Wasserstoff oder ein Rest der Formel -(CH₂)ₙCO₂R ist, worin n 1 oder 2 und R Alkyl mit 1 bis 18 Kohlenstoffatomen oder 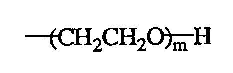 ist, worin m 1 bis 12 ist, R₁ mit Hydroxyl substituiertes und mit Sauerstoff unterbrochenes Alkyl mit 4 bis 18 Kohlenstoffatomen, -COR₄, worin R₄ Alkyl oder Alkenyl mit je 2 bis 12 Kohlenstoffatomen, 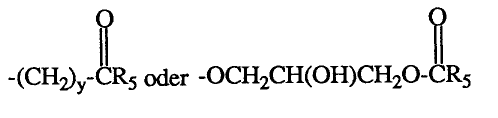 ist, oder R₁ 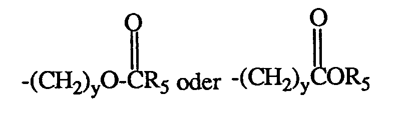 bedeutet, wobei R₅ Alkyl mit 1 bis 12 Kohlenstoffatomen oder Alkenyl mit 2 bis 12 Kohlenstoffatomen und y 1 bis 12 ist, und R₂ und R₃ unabhängig voneinander Wasserstoff, Alkyl mit 1 bis 12 Kohlenstoffatomen, Alkenyl mit 2 bis 12 Kohlenstoffatomen, -OR₁, worin R₁ die angegebene Bedeutung hat, oder Chlor sind, und R₆ Wasserstoff oder Alkyl mit 1 bis 4 Kohlenstoffatomen ist, ausgenommen die Verbindung, worin X O, R₀, R₂, R₃ und R₆ Wasserstoff und R₁ -CH₂-C(O)OC₂H₅ bedeutet.

2. Verbindungen nach Anspruch 1, worin R₂ und R₃ Wasserstoff oder Methyl sind.

3. Verbindungen nach Anspruch 1, worin R₁ mit Hydroxyl substituiertes und durch Sauerstoff unterbrochenes Alkyl mit 4 bis 12 Kohlenstoffatomen, -COR₄ oder 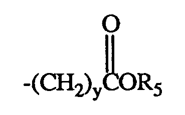 ist, worin R₄ Alkyl mit 4 bis 8 Kohlenstoffatomen, y 1 bis 4 und R₅ Alkyl mit 1 bis 4 Kohlenstoffatomen ist.

4. Verbindungen nach Anspruch 1, worin R₀ ein Rest der Formel -CH₂CH₂CO₂R ist, worin R Alkyl mit 1 bis 8 Kohlenstoffatomen oder 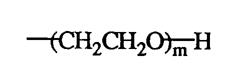 ist und m 6 bis 8 ist.

5. Verfahren zur Herstellung der Verbindungen der Formel (1) nach Anspruch 1, dadurch gekennzeichnet, dass man ein Gemisch aus den Verbindungen der Formeln 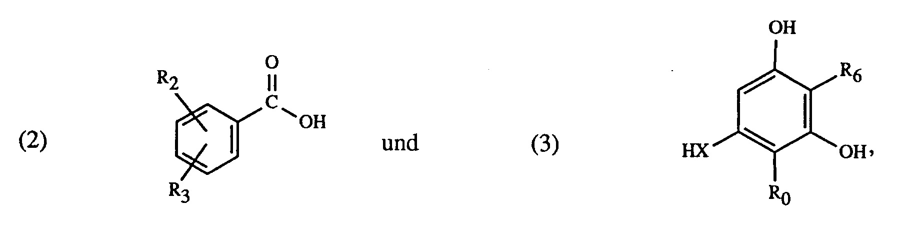 worin R₀, R₂, R₃, R₆ und X die in Anspruch 1 angegebene Bedeutung haben, erhitzt, die so erhaltene Zwischenverbindung der Formel 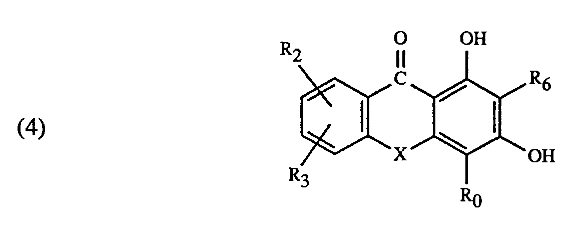 mit R₁-Cl oder R₁-Br, worin R₁ die in Anspruch 1 angegebene Bedeutung hat, zur Verbindung der Formel (1) umsetzt und isoliert.

6. Lichtempfindliches organisches Material, das als UV-Absorber mindestens eine Verbindung der Formel (1) und kein sterisch gehindertes Amin oder Hydroxyphenylbenztriazolderivat enthält.

7. Material nach Anspruch 6, dadurch gekennzeichnet, dass es ein Lack oder ein thermoplastisches Polymer ist.

8. Verfahren zum Schützen lichtempfindlicher organischer Materialien vor UV-Strahlung, dadurch gekennzeichnet, dass diese Materialien mit einer mindestens eine Verbindung der Formel (1) enthaltenden Schutzschicht, die frei von sterisch gehinderten Aminen oder Hydroxyphenylbenztriazolderivaten ist, versehen werden, oder dass in diese Materialien mindestens eine Verbindung der Formel (1), aber kein sterisch gehindertes Amin oder Hydroxyphenylbenztriazolderivat eingearbeitet wird.

## Claims

1. A compound of formula 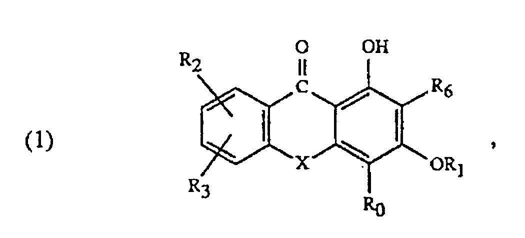 wherein X is O and R₀ is hydrogen or a radical of formula -(CH₂)ₙCO₂R, wherein n is 1 or 2 and R is alkyl of 1 to 18 carbon atoms or -(CH₂CH₂O)ₘ-H, wherein m is 1 to 12, R₁ is alkyl of 4 to 18 carbon atoms which is substituted by hydroxyl and interrupted by oxygen, or is -COR₄, wherein
R₄ is alkyl or alkenyl, each of 2 to 12 carbon atoms, 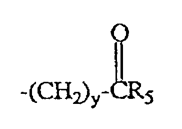 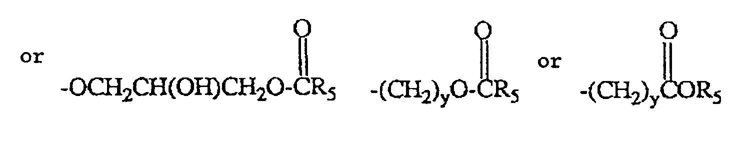 wherein
R₅ is alkyl
of 1 to 12 carbon atoms or alkenyl of 2 to 12 carbon atoms, and y is 1 to 12, and R₂ and R₃ are each independently of the other hydrogen, alkyl of 1 to 12 carbon atoms, alkenyl of 2 to 12 carbon atoms, -OR₁, wherein R₁ has the given meaning, or are chloro, and R₆ is hydrogen or alkyl of 1 to 4 carbon atoms, with the exception of the compound in which X is 0, R₀, R₂, R₃ and R₆ are hydrogen and R₁ is -CH₂-C(O)OC₂H₅.

2. A compound according to claim 1, wherein R₂ and R₃ are hydrogen or methyl.

3. A compound according to claim 1, wherein R₁ is alkyl of 4 to 12 carbon atoms which is substituted by hydroxyl and interrupted by oxygen, or is -COR₄ or 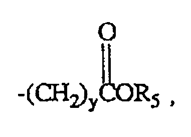 wherein R₄ is alkyl of 4 to 8 carbon atoms, y is 1 to 4, and R₅ is alkyl of 1 to 4 carbon atoms.

4. A compound according to claim 1, wherein R₀ is a radical of formula -CH₂CH₂CO₂R, wherein R is alkyl of 1 to 8 carbon atoms or -(CH₂CH₂O)ₘ-H and m is 6 to 8.

5. A process for the preparation of a compound of formula (1) according to claim 1, which comprises heating a mixture of the compounds of formulae 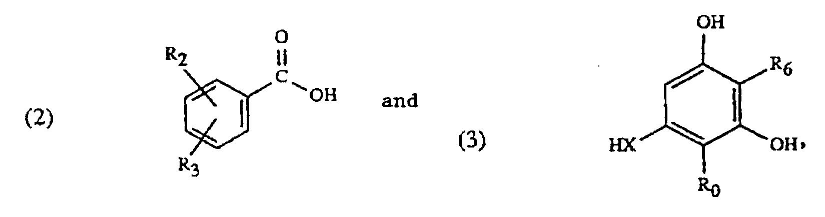 wherein R₀, R₂, R₃, R₆ and X are as defined in claim 1, reacting the intermediate thus obtained of formula 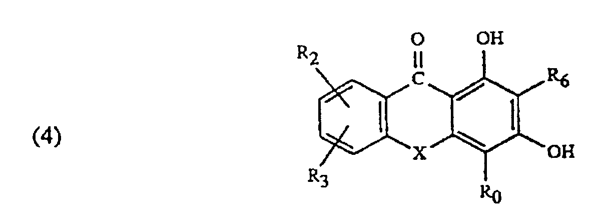 with R₁-Cl or R₁-Br, wherein R₁ is as defined in claim 1, to give the compound of formula (1) and isolating this compound.

6. A light-sensitive organic material which contains, as UV absorber, at least one compound of formula (1) and which does not contain a sterically hindered amine or hydroxyphenylbenzotriazole derivative.

7. A material according to claim 6, which is a coating composition or a thermoplastic polymer.

8. A method of protecting light-sensitive organic materials against UV radiation, which comprises providing said materials with a protective layer which contains at least one compound of formula (1) which is free from sterically hindered amines or hydroxyphenylbenzotriazole derivatives, or incorporating in said materials at least one compound of formula (1) but excluding any sterically hindered amine or hydroxyphenylbenzotriazole derivative.

## Revendications

1. Composés de formule : 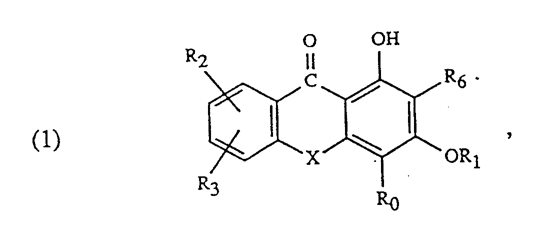 dans laquelle X représente l'oxygène et Rₒ l'hydrogène ou un radical de formule -(CH₂)ₙCO₂R, dans laquelle n vaut 1 ou 2 et R est un alkyle ayant de 1 à 18 atomes de carbone, ou un radical 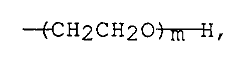 dans laquelle m vaut de 1 à 12, R₁ représente un hydroxyalkyle ayant de 4 à 18 atomes de carbone, interrompu par de l'oxygène, un radical -COR₄, dans lequel R₄ est un alkyle ou un alcényle avec chacun de 2 à 12 atomes de carbone, un radical 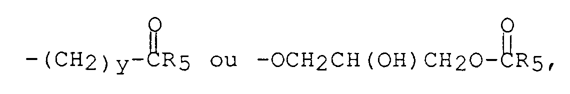 ou bien R₁ est un radical 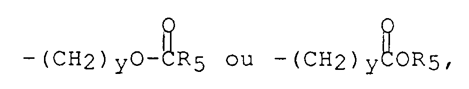 R₅ étant un alkyle avec de 1 à 12 atomes de carbone ou un alcényle avec de 2 à 12 atomes de carbone et y valant de 1 à 12, et
R₂ et R₃ représentent chacun, indépendamment l'un et l'autre, l'hydrogène, un alkyle avec de 1 à 12 atomes de carbone, un alcényle de 2 à 12 atomes de carbone, un groupe -OR₁, dans lequel R₁ a la signification donnée, ou le chlore, et R₆ représente l'hydrogène ou un alkyle ayant de 1 à 4 atomes de carbone, à l'exception du composé dans lequel X est l'oxygène, R_{O}, R₂, R₃ et R₆ signifie l'hydrogène et R₁ est le radical -CH₂-C(O)OC₂H₅.

2. Composés selon la revendication 1 dans lesquels R₂ et R₃ sont l'hydrogène ou le méthyle.

3. Composés selon la revendication 1 dans lesquels R₁ est un hydroxy-alkyle ayant de 4 à 12 atomes de carbone, interrompu par de l'oxygène, un radical -COR₄ ou 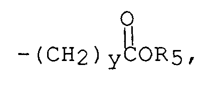 dans laquelle R₄ désigne un alkyle avec de 4 à 8 atomes de carbones, y vaut de 1 à 4 et R₅ est un alkyle avec de 1 à 4 atomes de carbone.

4. Composés selon la revendication 1, dans lesquels Rₒ est un radical -CH₂CH₂CO₂R, dans laquelle R est un alkyle ayant de 1 à 8 atomes de carbone, ou un radical -(CH₂CH₂O)-ₘH et m vaut de 6 à 8.

5. Procédé de préparation des composés de formule (1) selon la revendication 1, procédé caractérisé en ce qu'on chauffe un mélange des composés de formules 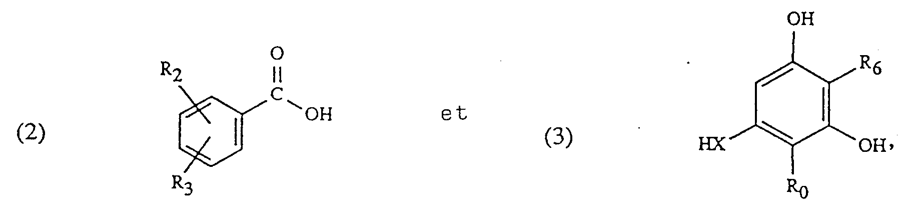 dans lesquelles Rₒ, R₂, R₃, R₆ et X ont les significations données dans la revendication 1 puis on fait réagir le composé intermédiaire ainsi obtenu de formule : 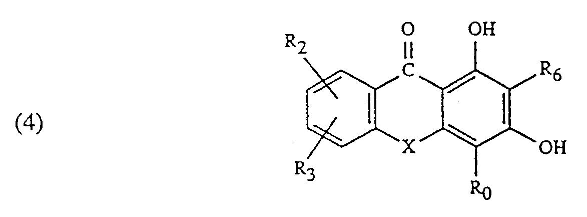 avec R₁-Cl ou R₁-Br, dans lesquels R₁ a la signification donnée dans la revendication 1 ce qui donne le composé de formule (1) que l'on isole.

6. Matière organique photosensible contenant comme absorbeurs d'UV au moins un composé de formule (1) et aucune amine à empêchement stérique ni de dérivés d'hydroxyphenylbenzotriazole.

7. Matière selon la revendication 6, caractérisée en ce qu'il s'agit d'une peinture ou d'un vernis ou d'un polymère thermoplastique.

8. Procède de protection de matières organiques photosensibles contre le rayonnement UV, procédé caractérisé en ce que l'on recouvre ces matières d'une couche protectrice contenant au moins un composé de formule (1), exempte d'amines à empêchement stérique ou de dérivés d'hydroxyphénylbenzotriazole, ou bien en ce qu'on leur incorpore au moins un composé de formule (1), mais sans aucune amine à empêchement stérique ou dérivé d'hydroxyphénylbenzotriazole.
